# EUROPÄISCHE PATENTSCHRIFT

(11) **EP 2 358 466 B1**
(45) Veröffentlichungstag und Bekanntmachung des Hinweises auf die Patenterteilung: **07.08.2013**
(21) Anmeldenummer: 09740067.5
(22) Anmeldetag: 19.10.2009
(51) Int. Cl.: B01F 7/00, C12M 1/02

(54) **RÜHRER FÜR BIOREAKTOR**
STIRRER FOR A BIOREACTOR
AGITATEUR POUR BIORÉACTEUR

(30) Priorität: 20.11.2008 DE 102008058338
(43) Veröffentlichungstag der Anmeldung: 24.08.2011
(73) Patentinhaber: Sartorius Stedim Biotech GmbH, 37079 Göttingen (DE)
(72) Erfinder: REIF, Oscar-Werner, 30173 Hannover (DE); FATHERAZI, Shahmir, 52066 Aachen (DE); GRELLER, Gerhard, 37085 Göttingen (DE)
(74) Vertreter: Schneider, Peter Christian
(86) Internationale Anmeldenummer: PCT/EP2009/007474
(87) Internationale Veröffentlichungsnummer: WO 2010/057555

(56) Entgegenhaltungen:
- US-A- 857 683
- US-A- 1 661 255

## Beschreibung

### Gebiet der Erfindung

Die Erfindung betrifft einen Rührer angeordnet in einem Bioreaktor, der als ein Behälter mit flexiblen Wänden ausgebildet ist, mit einer Mehrzahl gelenkig jeweils um eine Querachse verschwenkbarer miteinander verbundener Arme, die gemeinsam um eine Drehachse drehbar sind, wobei ein erster Arm mit seinem ersten Armende schwenkbar an einem ersten Achsteilende eines ersten um die Drehachse in einem feststehenden Basisteil drehbar gelagerten Achsteiles einer ersten Halterung angeordnet ist, die an einer ersten Seite des Behälters befestigt ist.

Rührer sind ein wesentlicher Teil von Bioreaktoren zum Homogenisieren von Temperatur- und Konzentrationsunterschieden. Außerdem verhindern die Rührer eine Zellabsetzung in Bioreaktoren und dispergieren die Luft bzw. Gase in begasten Bioreaktoren.

### Stand der Technik

Aus der US 2,045,710 A ist ein Rührer für einen Behälter bekannt, der aus mehreren gelenkig miteinander verbundenen Armen besteht, die im gestreckten Zustand durch eine Behälteröffnung in den Behälter eingeschoben werden können, wobei beim Berühren des Behälterbodens einige Schenkel nach außen öffnen. Ein erster Arm ist mit den restlichen mit ihm verbunden Armen gemeinsam um eine Drehachse drehbar. Dabei ist der erste Arm mit seinem ersten Armende drehbar an einer ersten Halterung angeordnet, die an einer ersten in vertikaler Richtung oberen Seite des Behälters befestigt ist.

Nachteilig bei dem bekannten Rührer ist, dass er mit seinen unteren beiden Armen beim Rotieren auf der unteren Seite, den Behälterboden, schleift. Bei einem Metallbehälter erzeugt dieses Schleifen unerwünschte Partikel, während bei einem Behälter aus Kunststofffolie die Folie nach kurzer Betriebszeit beschädigt werden würde. Ein waagerechter oder schräger Betrieb ist mit einem solchen Rührer nicht möglich.

Weiterhin ist aus der US 6,769,800 B1 ein klappbares Schikanensystem bzw. eine klappbare Lenkblechvorrichtung bekannt, die in einen Behälter oder Bioreaktor durch eine Öffnung einsetzbar ist, die kleiner ist als der Behälterdurchmesser. Die Schikanenanordnung ist aus mehreren Ringen und Längssegmenten bzw. Armen aufgebaut, wobei die Längssegmente nach dem Einschieben in den Behälter beim Aufsetzen ausgefahren werden und sich so an den Behälterwänden als Schikanen anlegen können. Die Schikanen wirken dann beim Rühren mit einem Rührer als Schikanen bzw. Strombrecher.

Weder ist diese Schikanevorrichtung zum Zusammenfalten mit einem flexiblen Behälter geeignet noch ist aus dieser Druckschrift ein Hinweis auf einen faltbaren Rührer zu entnehmen.

Aus der US 2002/0105856 A1 ist ein Rührer, gemäß dem Oberbegriff des Anspruchs 1, für Bioreaktoren bekannt, die insbesondere als Behälter mit flexiblen Wänden ausgebildet sind. Der Rührer weist einen flexiblen Rührschlauch auf, der an zwei gegenüberliegenden Reaktorwänden, die er durchstößt, in statischen Dichtungen statisch gehalten wird. Um den Rührschlauch zwischen seinen beiden fixierten Enden in einer Art rotierende Bewegung zu versetzen, wird in den Schlauch eine relativ starre, spiralisch bzw. schraubenförmig ausgebildete Antriebsachse eingeführt.

Weiterhin ist aus der US 857,683 A ein in einem Fass bzw. starren Behälter angeordneter Rührer bekannt. Der Rührer weist eine Mehrzahl gelenkig jeweils um eine Querachse verschwenkbarer miteinander verbundener Arme auf, die gemeinsam um eine Drehachse drehbar sind, wobei ein erster Arm mit seinem ersten Armende schwenkbar an einem im Behälterinnenraum zugewandten Ende einer Teleskophülse angeordnet ist. Die aus dem Behälterinnenraum herausführende Teleskophülse ist längsverschieblich mit einem um die gemeinsame Drehachse drehbaren Schaft verbunden. Der drehbare Schaft ist zwischen einer oberen Halterung bzw. Lager und einer unteren Halterung bzw. Lager drehbar angeordnet. Der dem ersten Arm abgewandte letztfolgende Arm ist mit seinem dem ersten Arm abgewandten zweiten Armende schwenkbar an dem der Halterung benachbarten Ende des Schaftes schwenkbar verbunden. Durch Herausziehen der Teleskophülse können die Arme eingeklappt, d.h. längs des Schaftes angeordnet werden. Beim Aus- und Einklappen der Arme wird die Teleskopschiene durch die Seitenwandung bzw. Decke des Behälters verschoben. Durch den starren Schaft muss der Abstand zwischen Behälterboden und Behälterdecke gleichbleiben.

Aus der US 1,661,255 A ist ein Rührer bekannt, der ebenfalls in einem Fass 1 bzw. starren Behälter angeordnet ist. Der Behälter weist am oberen Ende eine von einem Flansch umgebene Öffnung auf. Zentral weist der Behälter einen Stab auf, der mit einem Ende mit einer am Boden des Behälters zentral angeordneten Halterung befestigt ist. Das andere freie Ende des Stabes ragt in die Öffnung des Flansches hinein. Durch die Öffnung ist ein zweiarmiger Rührer anbringbar. Die beiden Arme sind über eine Querachse an ihren einander zugewandten Enden schwenkbar miteinander verbunden. Der Arm weist an seinem dem Arm abgewandten Ende, das gegenüber dem dem anderen Arm zugewandten Ende um 90° verdreht ist, einen Längsschlitz auf, in dem der Stab eingreift. Der Arm ist so ausgebildet, dass er mit einem dem zweiten Arm zugewandten unteren Teil parallel zur Wandung des Behälters geführt wird. Der obere Teil des Armes ist um 90° gegenüber dem ersten Teil verdreht und so gewölbt, dass er mit seinem freien Ende durch die Öffnung in den Flansch eingreift. An seinem freien Ende weist der Arm eine Bohrung auf, über die ein Werkzeug greifen kann, dass die Arme und um die Stange rotiert.

Dabei werden das freie Ende des Armes und das freie Ende der Stange in dem Flansch nicht geführt. Die Öffnung des Flansches weist einen solchen Durchmesser auf, dass der Arm zusammen mit dem verschwenkbaren Arm aus dem Behälter herausziehbar ist.

Der erste Arm, der als Kratzerarm am Boden des Behälters wirken soll, bezührt diesen.

### Aufgabenstellung

Aufgabe der vorliegenden Erfindung ist es daher, einen faltbaren Rührer zur Verfügung zu stellen, der raumsparend zusammenfaltbar ist und insbesondere in einen Behälter mit flexiblen Wänden eingesetzt werden kann.

### Darstellung der Erfindung

Diese Aufgabe wird in Verbindung mit dem Oberbegriff des Anspruches 1 dadurch gelöst, dass der dem ersten Arm abgewandte letztfolgende Arm mit seinem dem ersten Arm abgewandten zweiten Armende schwenkbar an einem ersten Achsteilende eines um die Drehachse in einem Basisteil drehbaren Achsteiles einer zweiten Halterung angeordnet ist, dass die zweite Halterung an einer der ersten seite des Behälters gegenüberliegenden zweiten Seite des Behälters befestigt ist, und dass die mit den Achsteilen gelenkig verbundenen Armenden jeweils um eine quer zur Drehachse angeordnete Querachse schwenkbar sind.

Durch die Anordnung von zwei an gegenüberliegenden Stirnseiten des Behälters angeordneten Halterungen wird ein unerwünschtes Schleifen an der in vertikaler Richtung unteren Seite des Behälters zuverlässig vermieden. Ein solcher Rührer funktioniert nicht nur in einer vertikalen sondern auch in einer schrägen oder waagerechten Position. Der Rührer passt sich an verschiedene Höhen eines Behälters an und wird beim Befüllen eines flexiblen Behälters mit zunehmendem Behältervolumen auseinander gezogen und geöffnet. Der Rührer funktioniert dabei sowohl im teilgeöffneten als auch im vollständig geöffneten Zustand.

Gemäß einer bevorzugten Ausführungsform der Erfindung sind zwei Arme vorgesehen, wobei der erste Arm mit seinem zweiten Armende mit dem ersten Armende des zweiten Armes, der den letztfolgenden Arm bildet, schwenkbar verbunden ist. Ein solcher Rührer ist einfach und kompakt aufgebaut und insbesondere für kleinere und mittlere Behälter geeignet.

Gemäß einer weiteren bevorzugten Ausführungsform der Erfindung sind zwei einander gegenüberliegende Armpaare vorgesehen, wobei der erste Arm jedes Armpaares mit seinem ersten Armende mit der ersten Halterung und mit seinem zweiten Armende mit dem ersten Armende des zweiten Armes, der den letztfolgenden Arm des Armpaares bildet, schwenkbar verbunden ist, und wobei der zweite Arm jedes Armpaares mit seinem zweiten Armende schwenkbar mit der zweiten Halterung verbunden ist. Ein solcher Rührer ist genauso flach zusammenfaltbar wie ein entsprechender Rührer mit nur einem Armpaar, weist aber eine doppelte Rührfläche auf.

Nach einer weiteren bevorzugten Ausführungsform der Erfindung sind drei Arme vorgesehen, wobei der zweite Arm mit seinem zweiten Armende mit dem ersten Armende des dritten Armes, der den letztfolgenden Arm bildet und dessen zweites Ende mit der zweiten Halterung in Verbindung steht, schwenkbar verbunden ist. Rührer mit drei oder mehr Armen sind insbesondere für mittelgroße und größere Behälter geeignet.

Nach einer weiteren bevorzugten Ausführungsform der Erfindung weist der zwischen ersten Arm und dritten Arm angeordnete zweite Arm als mittlerer Arm die doppelte Länge der anderen Arme auf. Dieser Rührer ist ebenfalls für größere Behälter geeignet. Zudem ist es günstig, dass der Rührer symmetrisch zur vertikalen Drehachse angeordnet ist.

Gemäß einer bevorzugten Ausführungsform der Erfindung weist die erste Halterung ein feststehendes Basisteil auf, das an der Innenfläche der ersten Seite des Behälters befestigt ist. Dabei weist die erste Halterung ein im Basisteil drehbar angeordnetes Achsteil auf, das an seinem dem Behälterinnenraum zugewandten ersten Achsteilende mit dem ersten Armende des ersten Armes schwenkbar verbunden ist und das an seinem dem Behälterinnenraum abgewandten zweiten Achsteilende mit einer Antriebsachse eines Antriebes verbindbar ist. Eine solche Verbindung zwischen Achteilende und Antriebsachse ist beispielsweise über eine Verbindungsbuchse, eine Verschraubung oder Verrastung möglich. Auch eine magnetische Ankupplung der Antriebsachse an das zweite Achsteilende ist möglich. Schließlich kann die Antriebsachse auch direkt in das zweite Achsteilende übergehen.

Gemäß einer weiteren bevorzugten Ausführungsform der Erfindung weist die zweite Halterung ebenfalls ein feststehendes Basisteil auf, das an der Innenfläche der zweiten Seite des Behälters, beispielsweise der Bodenfläche, befestigt ist. Die zweite Halterung weist dabei ein im Basisteil drehbar angeordnetes Achsteil auf, das an seinem dem Behälterinnenraum zugewandten ersten Achsteilende mit dem zweiten Armende des letzten Armes schwenkbar verbunden und mit seinem dem Behälterinnenraum abgewandten zweiten Achsteilende drehbar in dem Basisteil gelagert ist. Dabei ist es insbesondere von Vorteil, die Basisteile der Halterungen der mit ihnen jeweils benachbarten Seite des Behälters zu verschweißen. Für eine reibungsarme Rotation sind die Achsteile in einer bevorzugten Ausführungsform durch Gleitlager in den Basisteilen gelagert.

Nach einer weiteren bevorzugten Ausführungsform der Erfindung weist mindestens einer der Arme mindestens ein Rührblatt auf. Bei Verwendung zweier Arme weisen diese beispielsweise jeweils an ihren voneinander abgewandten Außenseiten jeweils zwei Rührblätter auf. Bei Verwendung dreier Arme kann der mittlere Arm, soweit er mindestens doppelt so lang ist wie die beiden anderen Arme auf seinen einander abgewandten Seiten jeweils vom Ende bis zur Mitte ein konvex gebogenes Rührblatt aufweisen. Ein Zusammenfalten in einer Ebene ist dann noch möglich.

Gemäß einer weiteren bevorzugten Ausführungsform der Erfindung ist ein Rührblatt einerseits schwenkbar an der Gelenkverbindung zwischen dem ersten Ende des letztfolgenden Armes und dem zweiten Ende des benachbarten Armes angelenkt und andererseits schwenkbar an ein erstes Ende eines Führungsstabes angelenkt, der mit seinem zweiten Ende schwenkbar an der zweiten Halterung angelenkt ist und das Rührblatt bei unterschiedlichen Abständen der Halterungen in vertikaler Richtung in gleicher horizontaler Lage hält. Entsprechend ist es bei zwei einander gegenüberliegenden Armpaaren möglich, dass jedes Armpaar ein verschwenkbares Rührblatt aufweist, das jeweils von einem Führungsstab in horizontaler Lage gehalten wird.

Nach einer weiteren bevorzugten Ausführungsform der Erfindung ist eine erste Blatthalterung mit einer Mehrzahl von Rührblättern einerseits schwenkbar an der Gelenkverbindung zwischen dem ersten Ende des letztfolgenden Armes und dem zweiten Ende des benachbarten Armes angelenkt und andererseits schwenkbar an ein erstes Ende eines Führungsstabes angelenkt, der mit seinem zweiten Ende schwenkbar an der zweiten Halterung angelenkt ist und die erste Blatthalterung mit den Rührblättern bei unterschiedlichen Abständen der Halterungen in vertikaler Richtung in gleicher horizontaler Lage hält. Durch die Mehrzahl von Rührblättern an der ersten Blatthalterung wird eine bessere und günstigere Durchmischung des zu rührenden bzw. zu mischenden Mediums erreicht. Zudem wird sichergestellt, dass bei unterschiedlichen Füllzuständen, in denen die Halterungen unterschiedliche Abstände zueinander aufweisen, die Halterung mit ihren Rührblättern in gleicher horizontaler Lage gehalten wird.

Nach einer weiteren bevorzugten Ausführungsform der Erfindung ist bei drei Armen eine zweite Blatthalterung mit einer Mehrzahl von Rührblättern einerseits schwenkbar an der Gelenkverbindung zwischen dem ersten Ende des zweiten Armes und dem zweiten Ende des benachbarten ersten Armes angelenkt und ist andererseits schwenkbar an ein erstes Ende eines zweiten Führungsstabes angelenkt, der mit seinem zweiten Ende schwenkbar an der ersten Blatthalterung angelenkt ist und die zweite Blatthalterung bei unterschiedlichen Abständen der Halterungen in vertikaler Richtung in gleicher horizontaler Lage hält. Dadurch ist es möglich, bei größeren Behältern in vertikaler Richtung zwei Blatthalterungen mit jeweils einer Mehrzahl von Rührblättern übereinander anzuordnen und jeweils in vertikaler Richtung in gleicher horizontaler Lage zu halten.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibungen der Zeichnungen

In den Zeichnungen zeigen:
- Figur 1:: eine räumliche Darstellung eines faltbaren Rührers mit gestrichelt angedeutetem flexiblen Behälter und gestrichelt angedeutetem Antrieb mit Antriebsache,
- Figur 2:: eine räumliche Darstellung eines faltbaren Rührers mit zwei Armen, die jeweils zwei seitlich angeordnete Rührblätter aufweisen,
- Figur 3:: eine räumliche Darstellung eines faltbaren Rührers mit vier Armen,
- Figur 4:: eine räumliche Darstellung eines faltbaren Rührers mit drei Armen, wobei der mittlere Arm zwei Rührblätter aufweist,
- Figur 5:: eine räumliche Darstellung eines faltbaren Rührers mit zwei Armen und einer Blatthalterung mit mehreren Rührblättern,
- Figur 6:: eine räumliche Darstellung des Rührers von Figur 5 in einer weiter auseinander gefalteten Stellung in verkleinerter Darstellung,
- Figur 7:: eine räumliche Darstellung des faltbaren Rührers von Figur 5 in einer komplett auseinander gefalteten Stellung in verkleinerter Darstellung,
- Figur 8:: eine räumliche Darstellung einer Halterung für Rührblätter mit einer außen liegenden Anlenkung für eine Führungsstange in vergrößerter Darstellung,
- Figur 9:: eine räumliche Darstellung einer Halterung für Rührblätter mit innen liegender Anlenkung einer Führungsstange in vergrößerter Darstellung,
- Figur 10:: eine räumliche Darstellung einer weiteren Halterung für Rührblätter mit außen liegender Anlenkung für eine Führungsstange in vergrößerter Darstellung,
- Figur 11:: eine räumliche Darstellung einer weiteren Halterung für Rührblätter mit einer innen liegenden Anlenkung für eine Führungsstange in vergrößerter Darstellung,
- Figur 12:: eine Seitenansicht eines faltbaren Rührers mit zwei einander gegenüberliegenden Armpaaren mit jeweils einem Rührblatt und angelenkter Führungsstange und
- Figur 13:: eine räumliche Darstellung eines faltbaren Rührers mit drei Armen und zwei Blatthalterungen mit mehreren Rührblättern.

### Beschreibung der Ausführungsbeispiele

Ein Rührer 1 besteht im Wesentlichen aus miteinander verbundenen Armen 2, 3, einer ersten Filterung 4 und einer zweiten Halterung 5.

Die erste Halterung 4 weist ein feststehendes Basisteil 6, in dem ein Achsteil 7 drehbar gelagert ist, auf. Das Achsteil 7 weist ein erstes Achsteilende 8 auf, das mit einem ersten Armende 9 um eine quer zu einer Drehachse 10 verlaufenden Querachse 11 schwenkbar verbunden ist. An seinem dem ersten Achsteilende 8 abgewandten zweiten Achsteilende 12 ist das Achsteil 7 mit einer Antriebsachse 13 eines Antriebes 14 verbindbar.

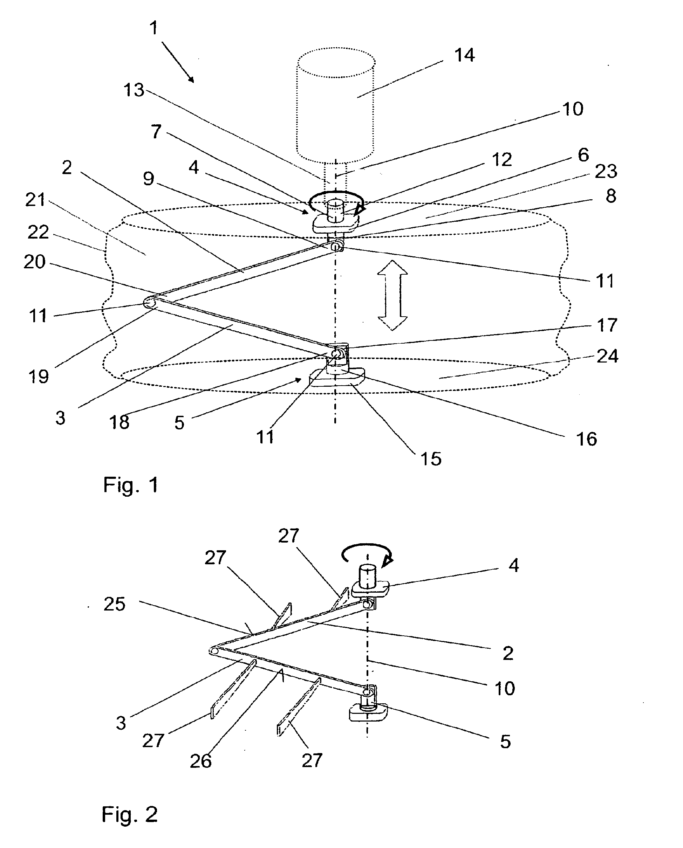

Die zweite Halterung 5 weist ein Basisteil 15 auf, in dem ein Achsteil 16 drehbar gelagert ist. Das Achsteil 16 ist an seinem der ersten Halterung 4 zugewandten ersten Achsteilende 17 mit einem zweiten Armende 18 des zweiten Armes 3 schwenkbar verbunden. Der zweite Arm 3 ist an seinem dem zweiten Armende 18 abgewandten ersten Armende 19 mit einem dem ersten Armende 9 abgewandten zweiten Armende 20 des ersten Armes 2 schwenkbar verbunden. Der zweite Arm 3 der Ausführungsbeispiele nach den Figuren 1, 2, 5, 6, 7 und 12 bildet dabei jeweils den letzten Arm der miteinander schwenkbar verbundenen Arme 2, 3.

Die Arme 2, 3 lassen sich mit den Achsteilen 7, 16 gemeinsam um die Drehachse 10 drehen.

Beim Einbau des Rührers 1 in einen Bioreaktor, der als Behälter 21 mit flexiblen Wänden, d.h. mindestens einer flexiblen Seitenwandung 22, einer ersten in vertikaler Richtung oben liegenden Seite 23 und einer in vertikaler Richtung unten liegenden zweiten Seite 24, ausgebildet ist, wird durch Verschweißen des Basisteiles 6 der ersten Halterung 4 mit der ersten Seite 23 und des zweiten Basisteiles 15 der zweiten Halterung 5 mit der zweiten Seite 24 in den Behälter befestigt.

Entsprechend dem Ausführungsbeispiel der Figur 2 weisen die Arme 2, 3 an ihren voneinander abgewandten Außenseiten 25, 26 jeweils zwei Rührblätter 27 auf.

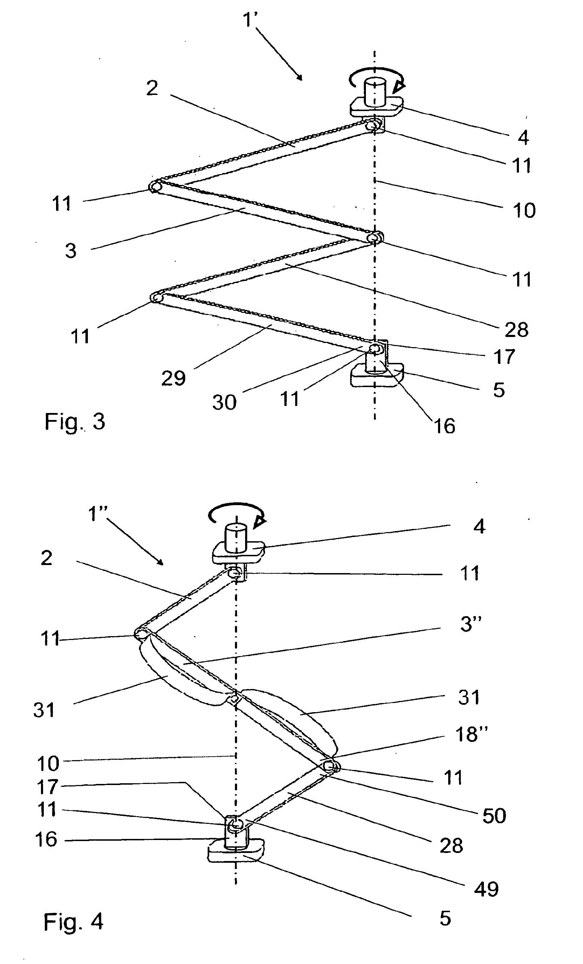

Das Ausführungsbeispiel der Figur 3 zeigt einen Rührer 1', der einen ersten Arm 2, einen zweiten Arm 3, einen dritten Arm 28 und einen vierten Arm 29 aufweist. Der vierte Arm 29 bildet dabei den letzten Arm, der mit seinem zweiten Armende 30 mit dem ersten Achsteilende 17 des Achsteiles 16 der zweiten Halterung 5 um die Querachse 11 schwenkbar verbunden ist.

Gemäß dem Ausführungsbeispiel der Figur 4 weist der Rührer 1" einen ersten Arm 2, einen zweiten Arm 3" und einen dritten Arm 28 auf. In diesem Ausführungsbeispiel bildet der dritte Arm 28 den letzten Arm, der mit seinem zweiten Ende 49 mit der zweiten Halterung 5 schwenkbar verbunden ist. Der zweite Arm 3" ist dabei doppelt so lang wie seine benachbarten Arme 2, 28. Der zweite Arm 3" trägt an seinen beiden einander abgewandten Außenseiten um seine halbe Länge versetzt jeweils ein konvex geformtes Rührblatt 31. Der dritte Arm 28 ist mit seinem ersten Ende 50 mit dem zweiten Ende 18" des zweiten Armes 3" schwenkbar verbunden.

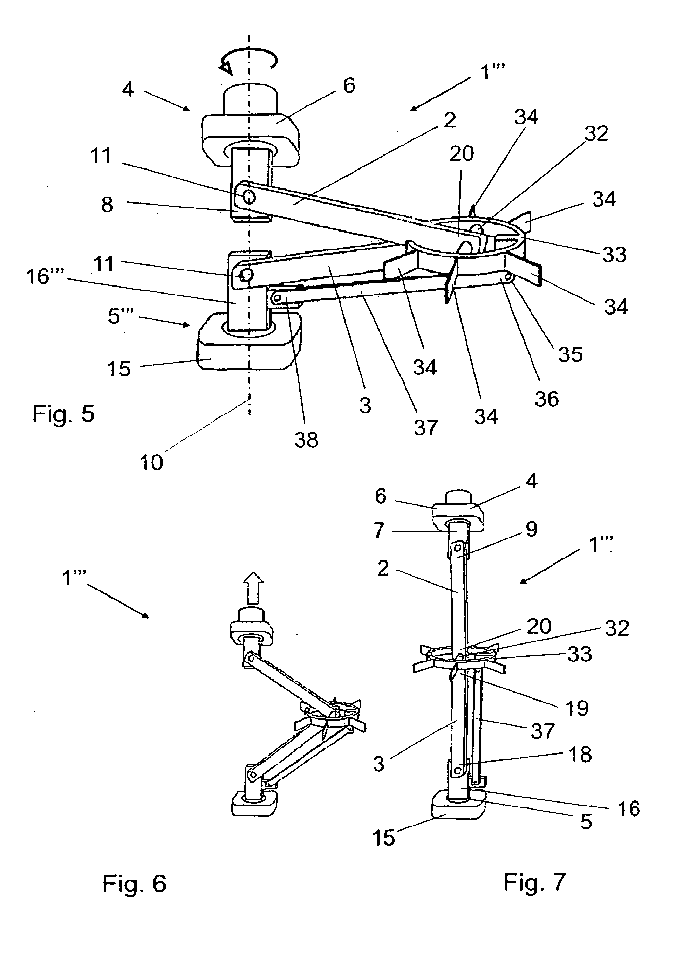

Das Ausführungsbeispiel der Figuren 5 bis 7 zeigt einen Rührer 1"', der an seiner Gelenkverbindung zwischen ersten Arm 2 und zweiten Arm 3 eine um eine Verbindungsachse 32 schwenkbare Blatthalterung 33 zur Befestigung mehrerer, im Ausführungsbeispiel sechs, Rührblätter 34 aufweist. Die Blatthalterung 33 weist eine zweite Anlenkstelle 35 auf, an der sie mit einem ersten Ende 36 eines Führungsstabes 37 schwenkbar angelenkt ist. Der Führungsstab 37 verläuft etwa parallel zu dem zweiten Arm 3 und ist mit seinem dem ersten Ende 36 abgewandten zweiten Ende 38 an dem Achsteil 16"' der zweiten Halterung 5'" angelenkt.

Figur 6 zeigt den Rührer 1"' in einer mittleren Stellung und Figur 7 zeigt den Rührer 1"' in einer komplett ausgezogenen bzw. auseinander gefalteten Stellung.

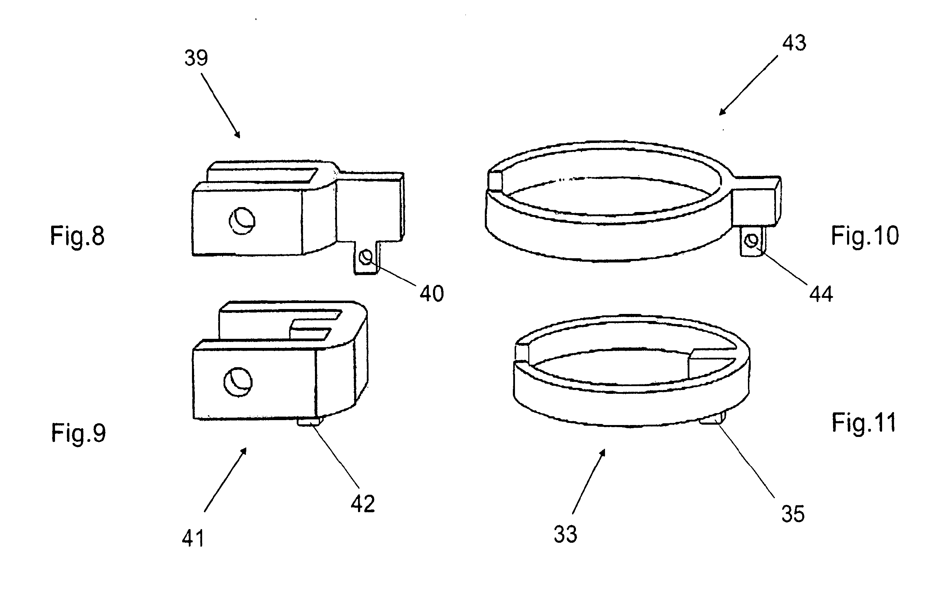

Figur 8 zeigt eine Blatthalterung 39 mit einer äußeren Anlenkstelle 40 zur Halterung eines nicht dargestellten Rührblattes.

Figur 9 zeigt eine weitere Blatthalterung 41 mit einer inneren Anlenkstelle 42.

Figur 10 zeigt eine weitere Ausführungsform einer Blatthalterung 43 mit einer äußeren Anlenkstelle 44 und Figur 11 zeigt die Blatthalterung 33 mit der inneren Anlenkstelle 35 entsprechend dem Ausführungsbeispiel der Figur 5 jedoch ohne Darstellung der Rührblätter.

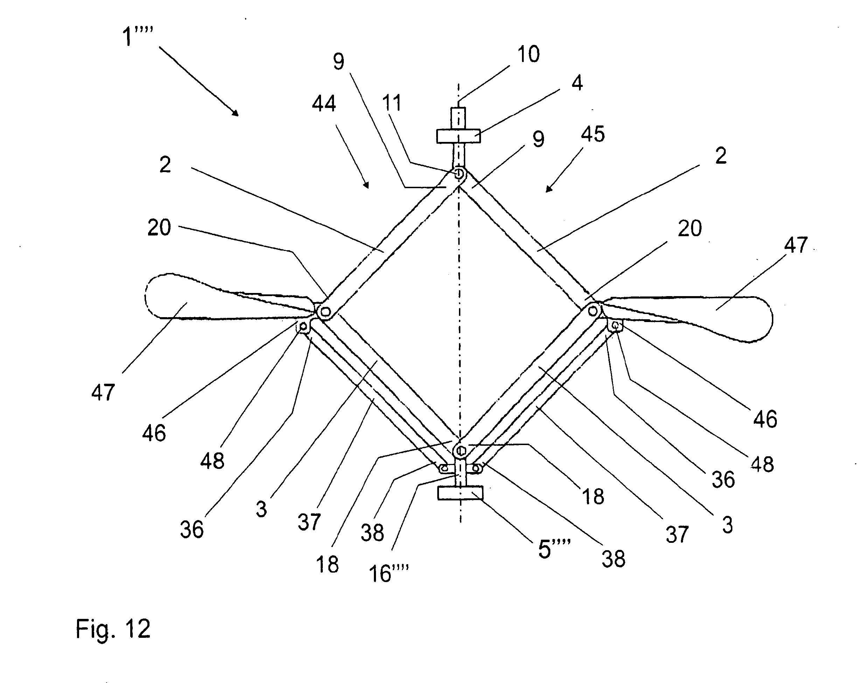

Das Ausführungsbeispiel der Figur 12 zeigt einen Rührer 1"" mit zwei einander gegenüberliegenden Armpaaren 44, 45. Jedes Armpaar 44, 45 weist an seiner Gelenkverbindung zwischen ersten Arm 2 und zweiten Arm 3 eine Blatthalterung 46 mit einem Rührblatt 47 auf. Die Blatthalterung 46 ist dabei über ihre Anlenkstelle 48 mit dem Führungsstab 37 verbunden, der mit seinem zweiten Ende 38 an dem Achsteil 16"" der zweiten Halterung 5"" schwenkbar angelenkt ist.

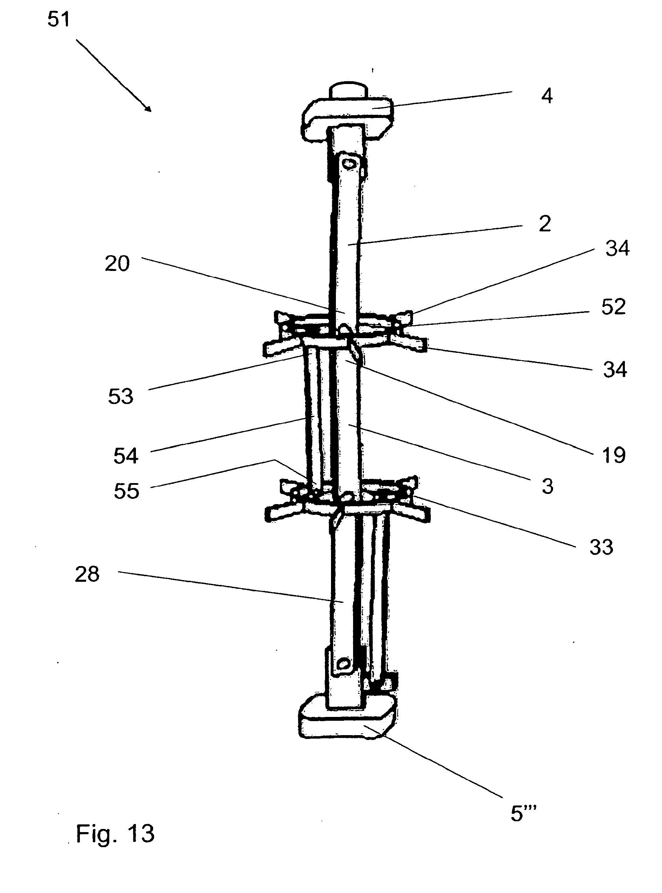

Das Ausführungsbeispiel der Figur 13 zeigt einen Rührer 51 mit drei Armen (2, 3, 28), der eine zweite Blatthalterung 52 für eine Mehrzahl von Rührblättern 34 aufweist, die einerseits schwenkbar an der Gelenkverbindung zwischen dem ersten Armende 19 des zweiten Armes 3 und dem zweiten Armende 20 des benachbarten ersten Armes 2 angelenkt ist. Andererseits ist die zweite Blatthalterung 52 schwenkbar an ein erstes Ende 53 eines zweiten Führungsstabes 54 angelenkt, der mit seinem zweiten Ende 55 schwenkbar an der ersten Blatthalterung 33 angelenkt ist und die zweite Blatthalterung 52 mit den Rührblättern 34 bei unterschiedlichen Abständen der Halterungen 4, 5"' in vertikaler Richtung in gleicher horizontaler Lage hält.

Damit ist allen Ausführungsformen der Erfindung gemeinsam, dass der in einem flexiblen Behälter 21 angeordnete Rührer 1, 1", 1"', 1"" zusammen mit dem Behälter 21 flach gefaltet werden kann und bei Befüllung des Behälters 21 mit diesem entfaltet wird.

## Patentansprüche

1. Rührer (1, 1', 1", 1"', 1"") angeordnet in einem Bioreaktor, der als ein Behälter (21) mit flexiblen Wänden (22) ausgebildet ist, mit einer Mehrzahl gelenkig jeweils um eine Querachse (11) verschwenkbarer miteinander verbundener Arme (2, 3, 28, 29), die gemeinsam um eine Drehachse (10) drehbar sind, wobei ein erster Arm (2) mit seinem ersten Armende (9) schwenkbar an einem ersten Achsteilende (8) eines ersten um die Drehachse (10) in einem feststehenden Basisteil (6) drehbar gelagerten Achsteiles (7) einer ersten Halterung (4) angeordnet ist, die an einer ersten Seite (23) des Behälters (21) befestigt ist,
**dadurch gekennzeichnet,**
**dass** der dem ersten Arm (2) abgewandte letztfolgende Arm (3, 28, 29) mit seinem dem ersten Arm (2) abgewandten zweiten Armende (18, 20, 30) schwenkbar an einem ersten Achsteilende (17) eines um die Drehachse (10) in einem Basisteil (15) drehbaren Achsteiles (16, 16''', 16'''') einer zweiten Halterung (5, 5'''', 5'''') angeordnet ist, dass die zweite Halterung (5, 5''', 5'''') an einer der ersten Seite (23) des Behälters (21) gegenüberliegenden zweiten Seite (24) des Behälters (21) befestigt ist, und dass die mit den Achsteilen (7, 16, 16''', 16'''') gelenkig verbundenen Armenden (9, 18, 30, 49) jeweils um eine quer zur Drehachse (10) angeordnete Querachse (11) schwenkbar sind.

2. Rührer nach Anspruch 1,
**dadurch gekennzeichnet,**
**dass** zwei Arme (2, 3) vorgesehen sind, wobei der erste Arm (2) mit seinem zweiten Armende (20) mit dem ersten Armende (19) des zweiten Armes (3), der den letztfolgenden Arm bildet, schwenkbar verbunden ist.

3. Rührer nach Anspruch 1,
**dadurch gekennzeichnet,**
**dass** zwei einander gegenüberliegende Armpaare (44, 45) vorgesehen sind, wobei der erste Arm (2) jedes Armpaares (44, 45) mit seinem ersten Armende (9) mit der ersten Halterung (4) und mit seinem zweiten Armende (20) mit dem ersten Armende (19) des zweiten Armes (3), der den letztfolgenden Arm des Armpaares (44, 45) bildet, schwenkbar verbunden ist, und wobei der zweite Arm (3) jedes Armpaares (44, 45) mit seinem zweiten Armende (18) schwenkbar mit der zweiten Halterung (5"") verbunden ist.

4. Rührer nach Anspruch 1,
**dadurch gekennzeichnet,**
**dass** drei Arme (2, 3", 28) vorgesehen sind, wobei der zweite Arm (3") mit seinem zweiten Armende (18") mit dem ersten Armende (50) des dritten Armes (28), der den letztfolgenden Arm bildet, schwenkbar verbunden ist.

5. Rührer nach Anspruch 4,
**dadurch gekennzeichnet,**
**dass** der zwischen dem ersten Arm (2) und dem dritten Arm (28) angeordnete zweite Arm (3") als mittlerer Arm die mindestens doppelte Länge der anderen Arme (2, 28) aufweist.

6. Rührer nach einem der Ansprüche 1 bis 5,
**dadurch gekennzeichnet,**
**dass** das feststehende Basisteil (6) der ersten Halterung (4) an der Innenfläche der ersten Seite (23) des Behälters (21) befestigbar ist und
**dass** das im Basisteil (6) drehbar angeordnete Achsteil (7) an seinem dem Behälterinnenraum abgewandten zweiten Achsteilende (12) mit einer Antriebsachse (13) eines Antriebes (14) verbindbar ist.

7. Rührer nach einem der Ansprüche 1 bis 6,
**dadurch gekennzeichnet,**
**dass** die zweite Halterung (5, 5"', 5"") das feststehende Basisteil (15) aufweist, das an der Innenfläche der zweiten Seite (24) des Behälters (21) befestigbar ist und
**dass** das Achsteil (16, 16"', 16"") mit seinem dem Behälterinnenraum abgewandten zweiten Achsteilende drehbar in dem Basisteil (15) gelagert ist.

8. Rührer nach Anspruch 6 oder 7,
**dadurch gekennzeichnet,**
**dass** die Basisteile (6, 15) der Halterungen (4, 5) mit den Seiten (23, 24) des Behälters (21) verschweißt sind.

9. Rührer nach einem der Ansprüche 6 bis 8,
**dadurch gekennzeichnet,**
**dass** die Achsteile (7, 16) durch Gleitlager in den Basisteilen (6, 15) gelagert sind.

10. Rührer nach Anspruch 9,
**dadurch gekennzeichnet,**
**dass** bei drei Armen (2, 3, 28) eine zweite Blatthalterung (52) für eine Mehrzahl von Rührblättern (34) einerseits schwenkbar an der Gelenkverbindung zwischen dem ersten Armende (19) des zweiten Armes (3) und dem zweiten Armende (20) des benachbarten ersten Armes (2) angelenkt und andererseits schwenkbar an ein erstes Ende (53) eines zweiten Führungsstabes (54) angelenkt ist, der mit seinem zweiten Ende (55) schwenkbar an der ersten Blatthalterung (33) angelenkt ist und die zweite Blatthalterung (52) mit den Rührblättern (34) bei unterschiedlichen Abständen der Halterungen (4, 5"') in vertikaler Richtung in gleicher horizontaler Lage hält.

11. Rührer nach einem der Ansprüche 1 bis 9,
**dadurch gekennzeichnet,**
**dass** mindestens einer der Arme (2, 3, 28, 29) mindestens ein Rührblatt (34) aufweist.

12. Rührer nach einem der Ansprüche 1 bis 9,
**dadurch gekennzeichnet,**
**dass** ein Rührblatt (47) einerseits schwenkbar an der Gelenkverbindung zwischen dem ersten Ende (19) des letztfolgenden Armes und dem zweiten Ende (20) des benachbarten Armes angelenkt und andererseits schwenkbar an ein erstes Ende (36) eines Führungsstabes (37) angelenkt ist, der mit seinem zweiten Ende (38) schwenkbar an der zweiten Halterung (5"") angelenkt ist und das Rührblatt (47) bei unterschiedlichen Abständen der Halterungen (4, 5"") in vertikaler Richtung in gleicher horizontaler Lage hält.

13. Rührer nach einem der Ansprüche 1 bis 9,
**dadurch gekennzeichnet,**
**dass** eine erste Blatthalterung (33) für eine Mehrzahl von Rührblättern (34) einerseits schwenkbar an der Gelenkverbindung zwischen dem ersten Armende (19) des letztfolgenden Armes und dem zweiten Ende (20) des benachbarten Armes angelenkt und andererseits schwenkbar an ein erstes Ende (36) eines Führungsstabes (37) angelenkt ist, der mit seinem zweiten Ende (38) schwenkbar an der zweiten Halterung (5"') angelenkt ist und die erste Blatthalterung (33) mit den Rührblättern (34) bei unterschiedlichen Abständen der Halterungen (4, 5"') in vertikaler Richtung in gleicher horizontaler Lage hält.

## Claims

1. A stirrer (1, 1', 1", 1'", 1"") arranged in a bioreactor that is embodied as a tank (21) having flexible walls (22), the stirrer comprising a plurality of articulately connected arms (2, 3, 28, 29) that are respectively pivotable about a transverse axis (11) and are jointly rotatable about a rotational axis (10), wherein a first arm (2) with its first arm end (9) is pivotably arranged on a first shaft end (8) of a first shaft (7) of a first mounting (4) attached to a first side (23) of the tank (21), said first shaft (7) being rotatably arranged in a fixed base part (6) and being rotatable about the rotational axis (10);
**characterized in that**
the last arm (3, 28, 29) facing away from the first arm (2) is pivotably disposed, via its second arm end (18, 20, 30) facing away from the first arm (2), on a first shaft end (17) of a shaft (16, 16", 16 "") of a second mounting (5, 5", 5""), said shaft (16, 16", 16"") being rotatable about the rotational axis (10) in a base part (15), and
the second mounting (5, 5"', 5"") is fastened to a second side (24) of the tank (21) that is opposite the first side (23) of the tank (21), and
the arm ends (9, 18, 19, 30, 49) articulately connected to the shafts (7, 16, 16", 16"") are respectively pivotable about a transverse axis (11) disposed transversely to the rotational axis (10).

2. A stirrer according to Claim 1,
**characterized in that**
two arms (2, 3) are provided, the first arm (2) being pivotably connected via its second arm end (20) to the first arm end (19) of the second arm (3), which forms the last arm.

3. A stirrer according to Claim 1,
**characterized in that**
two mutually opposed arm pairs (44, 45) are provided, the first arm (2) of each arm pair (44, 45) being pivotably connected via its first arm end (9) to the first mounting (4), and via its second arm end (20) to the first arm end (9) of the second arm (3), which forms the last arm of the arm pair (44, 45), and the second arm (3) of each arm pair (44, 45) being pivotably connected via its second arm end (18) to the second mounting (5"").

4. A stirrer according to Claim 1,
**characterized in that**
three arms (2, 3", 28) are provided, the second arm (3 ") being pivotably connected via its second arm end (18") to the first arm end (50) of the third arm (28), which forms the last arm.

5. A stirrer according to Claim 4,
**characterized in that**
the second arm (3") disposed between the first aim (2) and the third arm (28), being the centre arm, is at least twice as long as the other arms (2, 28).

6. A stirrer according to any of Claims 1 to 5,
**characterized in that**
the fixed base part of the first mounting (4) is attachable to the inner face of the first side (23) of the tank (21), and
that, at its second shaft end (12) facing away from the tank interior, the shaft (7) rotatably disposed in the base part (6) is connectable to a drive shaft (13) of a drive mechanism (14).

7. A stirrer according to any of Claims 1 to 6,
**characterized in that**
the second mounting (5, 5"', 5"") has a fixed base part (15) that is attachable to the inner face of the second side (24) of the tank (21), and
the shaft (16, 16"', 16"") is rotatably disposed in the base part (15) via its second shaft end that is facing away from the tank interior.

8. A stirrer according to either of Claims 6 or 7,
**characterized in that**
the base parts (6, 15) of the mountings (4, 5) are welded to the sides (23, 24) of the tank (21).

9. A stirrer according to any of Claims 6 to 8,
**characterized in that**
the shafts (7, 16) are supported on friction bearings in the base parts (6, 15).

10. A stirrer according to Claim 9,
**characterized in that**
in the case of three arms (2, 3, 28), a second blade mounting (52) for a plurality of stirring blades (34) is on the one hand pivotably coupled to the hinge joint between the first arm end (19) of the second arm (3) and the second arm end (20) of the adjacent first arm (2), and which blade mounting is on the other hand pivotably coupled to a first end (53) of a second guide rod (54), the second end (55) of which is pivotably coupled to the first blade mounting (33) and holds the second blade mounting (52) with the stirring blades (34) in the same horizontal orientation at different vertical distances between the mountings (4, 5"').

11. A stirrer according to any of Claims 1 to 9,
**characterized in that**
at least one of the arms (2, 3, 28, 29) has at least one stirring blade (34).

12. A stirrer according to any of Claims 1 to 9,
**characterized in that**
a stirring blade (47) is on the one hand pivotably coupled to the hinge joint between the first end (19) of the last arm and the second end (20) of the adjacent arm, and which stirring blade is on the other hand pivotably coupled to a first end (36) of a guide rod (37) that is pivotably coupled via its second end (38) to the second mounting (5"") and holds the stirring blade (47) in the same horizontal orientation at different vertical distances between the mountings (4, 5"").

13. A stirrer according to any of Claims 1 to 9,
**characterized in that**
a first blade mounting (33) for a plurality of stirring blades (34) is on the one hand pivotably coupled to the hinge joint between the first arm end (19) of the last arm and the second end (20) of the adjacent arm, and which first blade mounting is on the other hand pivotably coupled to a first end (36) of a guide rod (37) that is pivotably coupled via its second end (38) to the second mounting (5"') and holds the first blade mounting (33) with the stirring blades (34) in the same horizontal orientation at different vertical distances between the mountings (4, 5"').

## Revendications

1. Agitateur (1, 1', 1", 1"', 1 "") agencé dans un bioréacteur conçu sous forme de récipient (21) doté de parois flexibles (22), l'agitateur comprenant une pluralité de bras (2, 3, 28, 29) articulés les uns aux autres et pouvant tourner respectivement autour d'un axe transversal (11) et tourner ensemble autour d'un axe de rotation (10), un premier bras (2) doté d'une première extrémité de bras (9) étant agencé de façon pivotante sur une première extrémité d'arbre (8) d'un premier arbre (7) d'un premier montage (4) fixé à un premier côté (23) du récipient (21), ledit premier arbre (7) étant agencé de façon pivotante dans une partie base fixe (6) et pouvant tourner autour de l'axe de rotation (10); **caractérisé en ce que**
le dernier bras (3, 28, 29) opposé au premier bras (2) est agencé de façon pivotante, par l'intermédiaire de sa seconde extrémité de bras (18, 20, 30) opposée au premier bras (2), sur une première extrémité d'arbre (17) d'un arbre (16, 16", 16"") d'un second montage (5, 5", 5""), ledit arbre (16, 16", 16"") pouvant tourner autour de l'axe de rotation (10) dans une partie base (15), et
le second montage (5, 5 ", 5 "") est fixé à un second côté (24) du récipient (21) qui est opposé au premier côté (23) du récipient (21), et
les extrémités de bras (9, 18, 19, 30, 49) articulées sur les arbres (7, 16, 16", 16"") peuvent tourner respectivement autour d'un axe transversal (11) agencé de façon transversale à l'axe de rotation (10).

2. Agitateur selon la revendication 1,
**caractérisé en ce que**
deux bras (2, 3) sont prévus, le premier bras (2) étant relié de façon pivotante, par l'intermédiaire de sa seconde extrémité de bras (20), à la première extrémité de bras (19) du second bras (3), qui forme le dernier bras.

3. Agitateur selon la revendication 1,
**caractérisé en ce que**
deux paires de bras mutuellement opposées (44, 45) sont prévues, le premier bras (2) de chaque paire de bras (44, 45) étant relié de façon pivotante par l'intermédiaire de sa première extrémité de bras (9) au premier montage (4), et par l'intermédiaire de sa seconde extrémité de bras (20) à la première extrémité de bras (19) du deuxième bras (3), qui forme le dernier bras de la paire de bras (44, 45), et le deuxième bras (3) de chaque paire de bras (44, 45) étant relié de façon pivotante par l'intermédiaire de sa seconde extrémité de bras (18) au second montage (5"").

4. Agitateur selon la revendication 1,
**caractérisé en ce que**
trois bras (2, 3 ", 28) sont prévus, le deuxième bras (3") étant relié de façon pivotante par l'intermédiaire de sa seconde extrémité de bras (18") à la première extrémité de bras (50) du troisième bras (28), qui forme le dernier bras.

5. Agitateur selon la revendication 4,
**caractérisé en ce que**
le deuxième bras (3 ") agencé entre le premier bras (2) et le troisième bras (28) en tant que bras central est au moins deux fois plus long que les autres bras (2, 28).

6. Agitateur selon l'une quelconque des revendications 1 à 5,
**caractérisé en ce que**
la partie base fixe du premier montage (4) peut être fixée à la face intérieure du premier côté (23) du récipient (21), et
l'arbre (7) agencé de façon pivotante dans la partie base (6) peut être relié, sur sa deuxième extrémité d'arbre (12) opposée à l'intérieur du récipient, à un arbre d'entrainement (13) d'un mécanisme d'entrainement (14).

7. Agitateur selon l'une quelconque des revendications 1 à 6,
**caractérisé en ce que**
le second montage (5, 5 ", 5"") comporte la partie base fixe (15) pouvant être fixée à la face intérieure d'un second côté (24) du récipient (21), et
l'arbre (16, 16"', 16"") est agencé de façon pivotante dans la partie base par l'intermédiaire de sa seconde extrémité d'arbre qui est opposée à l'intérieur de récipient.

8. Agitateur selon l'une quelconque des revendications 6 ou 7,
**caractérisé en ce que**
les parties base (6, 15) des montages (4, 5) sont soudées aux côtés (23, 24) du récipient (21).

9. Agitateur selon l'une quelconque des revendications 6 à 8,
**caractérisé en ce que**
les arbres (7, 16) sont montés sur des paliers lisses dans les parties base (6, 15).

10. Agitateur selon la revendication 9,
**caractérisé en ce que**
en présence de trois bras (2, 3, 28), un second montage de pales (52) destiné à une pluralité de pales d'agitation (34) est, d'une part, couplé de façon pivotante à la charnière entre la première extrémité de bras (19) du deuxième bras (3) et la seconde extrémité de bras (20) du premier bras voisin (2), et, d'autre part, couplé de façon pivotante à une première extrémité (53) d'une seconde tige de guidage (54), dont la seconde extrémité (55) est couplée de façon pivotante au premier montage de pales (33) et maintient le second montage de pales (52) doté de pales d'agitation (34) dans la même orientation horizontale à des distances verticales différentes entre les montages (4, 5"').

11. Agitateur selon l'une quelconque des revendications 1 à 9,
**caractérisé en ce que**
au moins l'un des bras (2, 3, 28, 29) comporte au moins une pale d'agitation (34).

12. Agitateur selon l'une quelconque des revendications 1 à 9,
**caractérisé en ce que**
une pale d'agitation (47) est, d'une part, couplée de façon pivotante à la charnière entre la première extrémité (19) du dernier bras et la seconde extrémité (20) du bras voisin, et, d'autre part, couplée de façon pivotante à une première extrémité (36) d'une tige de guidage (37) qui est couplée de façon pivotante par l'inteimédiaire de sa seconde extrémité (38) au second montage (5 "") et maintient la pale d'agitation (47) dans la même orientation horizontale à des distances verticales différentes entre les montages (4, 5"").

13. Agitateur selon l'une quelconque des revendications 1 à 9,
**caractérisé en ce que**
un premier montage de pales (33) destiné à une pluralité de pales d'agitation (34) est, d'une part, couplé de façon pivotante à la charnière entre la première extrémité de bras (19) du dernier bras et la seconde extrémité (20) du bras voisin, et, d'autre part, couplé de façon pivotante à une première extrémité (36) d'une tige de guidage (37) qui est couplée de façon pivotante par l'intermédiaire de sa seconde extrémité (38) au second montage (5"') et maintient le premier montage de pales (33) doté des pales d'agitation (34) dans la même orientation horizontale à des distances verticales différentes entre les montages (4, 5"').
